Europäisches Patentamt

European Patent Office

Office européen des brevets

(19)

(11) Veröffentlichungsnummer: **0 168 428**

**B1**

(12) # EUROPÄISCHE PATENTSCHRIFT

(45) Veröffentlichungstag der Patentschrift:
23.08.89

(21) Anmeldenummer: 85900460.8

(22) Anmeldetag: 21.12.84

(86) Internationale Anmeldenummer:
PCT/EP 84/00421

(87) Internationale Veröffentlichungsnummer:
WO 85/02998 (18.07.85 Gazette 85/16)

(51) Int. Cl.⁴: **A 61 K 7/09**

(54) **VERFAHREN ZUR DAUERHAFTEN VERFORMUNG VON MENSCHLICHEN HAAREN.**

(30) Priorität: 14.01.84 DE 3401221

(43) Veröffentlichungstag der Anmeldung:
22.01.86 Patentblatt 86/4

(45) Bekanntmachung des Hinweises auf die Patenterteilung:
23.08.89 Patentblatt 89/34

(84) Benannte Vertragsstaaten:
FR GB NL

(56) Entgegenhaltungen:
EP-A-0 065 175
US-A-3 103 468

(73) Patentinhaber: GOLDWELL GMBH, CHEMISCHE FABRIK H.E. DOTTER, Zerninstrasse 10- 18, D-6100 Darmstadt- Eberstadt (DE)

(72) Erfinder: HEINZ, Dieter, Merianstrasse 15, D-6095 Gustavsburg 1 (DE)
Erfinder: KINGETER, Siegfried, Henry- Dunant- Strasse 15, D-6110 Dieburg (DE)
Erfinder: ROSE, Burkhard, Katharinenstrasse 14, D-6100 Darmstadt- Eberstadt (DE)
Erfinder: SEGAWA, Hirotsugu, 37, 2-chome Nagahoribashisuji, Minami- ku Osaka (JP)
Erfinder: TENNIGKEIT, Jürgen, Felsbergstrasse 51, D-6104 Seeheim 2 (DE)

(74) Vertreter: Helber, Friedrich G., Dipl.- Ing., Patentanwälte Dipl.- Ing. F. G. Helber Dipl.- Ing. J.K. Zenz Giesser Weg 47, D-6144 Zwingenberg (DE)

**Beschreibung**

Die Erfindung betrifft ein Verfahren zur dauerhaften Verformung von menschlichem Haar, bei dem das partieweise auf Wickler aufgerollte Haar mit einem konsistenzgebende(n) Zusatzstoff (e) enthaltenden haarkeratinreduzierenden Dauerverformungsmittel behandelt wird, welches bei Raumtemperatur (20°C) eine Viskosität von wenigstens 100 mPa.s hat, und welches nach einer ausreichenden Einwirkungszeit vom Haar abgespült und das Haar anschließend mit einer oxidierend wirkenden Lösung behandelt wird.

Für die dauerhafte Verformung von menschlichen Haaren, d.h. das sogenannte "Dauerwellen", werden bis heute überwiegend noch flüssige Dauerverformungsmittel verwendet, obwohl zum Teil auch bereits cremeformige oder beim Auftragen schaumförmige Mittel vorgeschlagen und verwendet wurden und werden. Bei der Anwendung der flüssigen Dauerverformungsmittel werden im allgemeinen zwei verschiedene Anwendungsweisen verfolgt. Entweder wird das in der Regel zuvor geschwaschene, handtuchtrockene Haar zunächst in mehrere Partien aufgeteilt, die dann mit einem Teil des flüssigen Dauerverformungsmittels vorgefeuchtet und anschließend auf Wickler aufgewickelt werden. Nach Beendigung des Wickelvorgangs werden die Wickel mit dem Rest des flüssigen Dauerverformungsmittels nachgefeuchtet. Bei der anderen Verfahrensweise wird das gewaschene und handtuchtrockene Haar ebenfalls in mehrere Partien aufgeteilt und dann ohne Vorfeuchtung mit einem Dauerverformungsmittel auf Wickel aufgewickelt. Die fertig aufgewickelten Haare werden dann mit der gesamten erforderlichen Menge des Dauerverformungsmittels gründlich durchfeuchtet. Nach Ablauf der erforderlichen Einwirkungszeit des Haarverformungsmittels wird das gewickelte Haar mit Wasser gespült und mit der wässrigen Lösung eines Oxidationsmittels fixiert. Da bei der Anwendung flüssiger Dauerverformungsmittel bereits behandelte Haarpartien optisch nicht von unbehandelten Haarpartien zu unterscheiden sind, sind Unter- oder Überdosierungen des Dauerverformungsmittels in einzelnen Haarpartien mit den sich daraus ergebenden Nachteilen nicht auszuschließen. Im ersteren Fall tritt eine unzureichende Verformung der betreffenden Haarpartien ein, während im letzteren Fall das flüssige Verformungsmittel auf die Kopfhaut und das Gesicht der zu behandelnden Person laufen und zu Hautreizungen führen kann. Bei einem bekannten zweistufigen Dauerwell-Verfahren (EP-A-0 065 175) wird nur in der ersten Verfahrensstufe ein flüssiges, schwächer verformungswirksames, in der zweiten Verfahrensstufe dagegen ein konsistentes, d.h. creme- oder gelförmiges, stärker verformungswirksames Dauerverformungsmittel auf das gewickelte Haar aufgetragen. Das flüssige Mittel benetzt die Haare insgesamt, wobei es - infolge seiner geringeren Verformungswirksamkeit - die empfindlichen Haarspitzen nicht schädigt. Auf die Kopfhaut ablaufendes flüssiges Dauerverformungsmittel hat auch eine verringerte Tendenz zur Verursachung von Hautreizungen. Das in der zweiten Verfahrensstufe aufgetragene konsistente Dauerverformungsmittel wirkt dagegen mehr oder weniger nur auf die in den Haarwickeln außen liegenden, d.h. haaransatznäheren, Haarpartien ein und tropft infolge seiner Konsistenz nicht auf die Kopfhaut ab, trägt aber auch nicht oder nur in verringertem Maße zur Verformung der in den Haarwickeln innenliegenden, d.h. haaransatzferneren Haarpartien bei.

Demgegenüber liegt der Erfindung die Aufgabe zugrunde, ein einstufiges Verfahren zur Dauerverformung von menschlichen Haaren unter Verwendung eines Dauerverformungsmittels mit verringerter Neigung zum Abtropfen oder Ablaufen anzugeben, welches eine zuverlässige und vom Haaransatz bis zu den empfindlichen Haarspitzen gleichmäßige Umformung des Haars ermöglicht.

Ausgehend von einem Verfahren der eingangs erwähnten Art wird diese Aufgabe erfindungsgemäß dadurch gelöst, daß nur ein Dauerverformungs mittel verwendet wird, welches bei Erwärmung auf eine Temperatur innerhalb eines Temperaturbereichs zwischen 25°C und 42°C einen deutlichen Viskositätsabfall von über 60 %, bezogen auf die Ausgangsviskosität, und auf einen Wert unter 50 mPa.s zeigt, daß das Dauerverformungsmittel nur auf in den Wickeln außen liegenden Haarpartien der ohne Dauerverformungsmittel aufgewickelten Haare aufgetragen wird, und daß die Viskosität des Dauerverformungsmittels dann durch die Erhöhung der Temperatur in einen Bereich zwischen 25°C und 42°C so weit verringert wird, daß es dünnflüssig wird und in die aufgewickelten Haarpartien eindringt und sie durchnetzt. Infolge der beim Auftragen viskosen oder cremeförmigen Konsistenz kann das Dauerverformungsmittel nicht abtropfen und ablaufen und die Kopfhaut benetzen, bzw. sogar ins Gesicht laufen. Selbst dann, wenn das erfindungsgemäß eingesetzte Dauerverformungsmittel beim späteren Erwärmen dünnflüssig wird, dringt es zunächst in die im Wickel inneren Haarpartien ein und benetzt diese. Nur bei Überdosierung, die aber bei vorschriftsmäßiger Anwendung vermeidbar ist, tritt dann überschüssiges Verformungsmittel noch bis zur Kopfhaut durch. Im Vergleich zu den üblichen Flüssig-Dauerwellen wird die Behandlung also als wesentlich komfortabler empfunden. Ein weiterer Vorteil des in der erfindungsgemäßen Weise auftragbaren Dauerverformungsmittel liegt darin, daß es infolge seiner Konsistenz auf der Außenseite der Haare sichtbar ist und deshalb eine gleichmäßige Auftragung auf sämtlichen Haarpartien ohne Über- oder Unterdosierung ermöglicht und somit auch zu einem gleichmäßigen Wellbild führt.

Wenn das Dauerverformungsmittel durch Wahl geeigneter Zusatzstoffe so eingestellt ist, daß die Viskositätserniedrigung bei Temperaturen unterhalb von etwa 30°C erfolgt, genügt zur Erhöhung der Temperatur des Dauerverformungsmittels die Körperwärme der zu behandelnden Person, wobei eine Abdeckung der aufgewickelten Haare nach der Aufbringung des Dauerverformungsmittels mit einer Wärmeverluste verhindernden Haube zweckmäßig sein kann. Bei dieser Verfahrensweise erfolgt die Viskositätserniedrigung des Dauerverformungsmittels relativ langsam, so daß auch die Durchfeuchtung der Haarwickel zeitverzögert von außen nach innen erfolgt. Die Einwirkungszeit auf die äußeren Wickellagen, d.h.

2

den Haaransatz, ist also höher und somit die Einwirkung stärker, als auf die empfindlicheren Haarspitzen.

Wenn die Viskositätserniedrigung des Dauerverformungs mittels dagegen erst bei höheren Temperaturen auftritt, kann die erforderliche Temperaturerhöhung mittels Wärmestrahlung, z. B. durch Infrarotstrahler, oder durch Aufblasen von erwärmter Luft, beispielsweise mit der in Friseursalons üblicherweise verwendeten Trockenhaube, bewirkt werden.

Bei dem erfindungsgemäßen Verfahren wird zweckmäßig ein Dauerverformungsmittel verwendet, bei dem der bzw. die konsistenzgebende(n) Zusatzstoff(e) in an sich bekannter Weise einen oder mehrere Paraffin(e), Petrolatum (Vaseline®), Ceresin(e), Fettalkohol(e), Fettsäure(n), Wachs(e), Polyglykol(e), Polyglykolester, Polyglykoläther, Aminoxid(e), Lanolinderivat(e) und/oder Ester und Äther ein- oder mehrwertiger organischer Säuren mit ein- oder mehrwertigen Alkoholen enthält bzw. enthalten.

Nachstehend werden vier Ausführungsbeispiele von in Verbindung mit dem erfindungsgemäßen Verfahren anwendbaren Dauerverformungsmitteln näher erläutert, deren Rezepturen zunächst in der nachstehendn listenförmigen Zusammenstellung wiedergegeben sind.

## Beispiele

| | 1 | 2 | 3 | 4 |
|---|---|---|---|---|
| Cetylalkohol | -- | -- | 0,50g | -- |
| Myristylalkohol | 0,80g | 0,60g | 0,40g | 0,70g |
| Oleylalkohol 92 - 96 | 0,30g | 0,60g | -- | 0,15g |
| Paraffinöl (dünnfl.) | -- | -- | -- | 1,00g |
| Petrolatum (Vaseline®) | 1,00g | 1,00g | 1,00g | -- |
| PEG-40 hydr.Ricinusöl | 1,00g | 0,75g | 0,75g | 0,30g |
| Cetylstearylalkohol-25EO (Ceteareth-25) | 0,50g | 0,35g | 0,50g | 0,30g |
| Parfümöl | 0,40g | 0,40g | 0,40g | 0,40g |
| Propylenglykol (1,3) | 1,50g | 1,50g | 1,50g | 1,50g |
| Cetyltrimetyl-ammonumchlorid | 0,10g | 0,10g | 0,10g | 0,10g |
| Alkyldimethylbetain 30 | -- | -- | -- | 1,00g |
| Thioglykolsäure 80 % | 12,50g | 12,50g | 12,50g | 10,00g |
| Ammoniak 25 % | 7,35g | 7,35g | 7,35g | 7,50g |
| Ammoniumhydrogencarbonat | 4,00g | 4,00g | 4,00g | 2,00g |
| Wasser, demineralisiert ad | 100ml | 100ml | 100ml | 100ml |

Bei den nach den vorstehenden Rezepturen zusammengestellten Dauerverformungsmitteln wurden die Viskositätswerte (gemessen mit einem Brookfield-Rotationsviskosimeter mit Spindel 3 und einer Umdrehungszahl von 5 U/min.) in Abhängigkeit von verschiedenen Temperaturen innerhalb des hier interessierenden Temperaturbereichs gemessen.

## Beispiel 1

Bei 20°C wurde eine Viskosität von 1350 mPa.s gemessen, die bei 30°C auf 20 mPa.s und bei 40°C auf weniger als 5 mPa.s abgefallen war. Der Schmelzbereich des Dauerverformungsmittels lag bei 26°C bis 27°C, so daß es für eine Dauerwellbehandlung von normalen Haaren ohne äußere Wärmezufuhr geeignet ist.

## Beispiel 2

Bei diesem Beispiel wurde bei 20°C eine Viskosität von 710 mPa.s gemessen, die bei 30°C auf 10 mPa.s und bei 40°C auf weniger als 5 mPa.s abgefallen war. Auch in diesem Fall liegt der Schmelzbereich von 25°C bis 27°C so niedrig, daß das Mittel für eine Anwendung ohne äußere Wärmezufuhr geeignet ist.

## Beispiel 3

Bei diesem Beispiel wurde eine Viskosität von 2800 mPa.s bei 20°C gemessen, die bei 40°C auf 50 mPa.s abgesunken war. Das Dauerverformungsmittel, dessen Schmelzbereich zwischen 37°C und 40°C liegt, ist also für eine Behandlung mit zusätzlicher Wärmeeinwirkung durch Wärmestrahlen oder Aufblasen erwärmter Luft geeignet.

**Beispiel 4**

Bei diesem Beispiel, dessen Rezeptur speziell für gefärbtes Haar entwickelt wurde, beträgt die Ausgangsviskosität bei 20°C 2600 mPa.s, bei 30°C 900 mPa.s und bei 40°C weniger als 5 mPa.s. Der bei etwa 31° bis 33°C liegende Schmelzbereich legt eine zusätzliche Wärmezufuhr bei der Behandlung nahe.

**Patentansprüche**

1. Verfahren zur dauerhaften Verformung von menschlichem Haar, bei dem das partieweise auf Wickler aufgerollte Haar mit einem konsistenzgebende(n) Zusatzstoff(e) enthaltenden haarkeratinreduzierenden Dauerverformungsmittel behandelt wird, welches bei Raumtemperatur (20°C) eine Viskosität von wenigstens 100 mPa.s hat, und welches nach einer ausreichenden Einwirkungszeit vom Haar abgespült und das Haar anschließend mit einer oxidierend wirkenden Lösung behandelt wird,
dadurch gekennzeichnet,
daß nur ein Dauerverformungsmittel verwendet wird, welches bei Erwärmung auf eine Temperatur innerhalb eines Temperaturbereichs zwischen 25°C und 42°C einen deutlichen Viskositätsabfall von über 60 %, bezogen auf die Ausgangsviskosität, und auf einen wert unter 50 mPa.s zeigt, daß das Dauerverformungsmittel nur auf in den Wickeln außen liegenden Haarpartien der ohne Dauerverformungsmittel aufgewickelten Haare aufgetragen wird, und daß die Viskosität des Dauerverformungsmittels dann durch die Erhöhung des Temperatur in einen Bereich zwischen 25° und 42°C soweit verringert wird, daß es dünnflüssig wird und in die aufgewickelten Haarpartien eindringt und sie durchnetzt.

2. Verfahren nach Anspruch 1, dadurch gekennzeichnet, daß zur Erhöhung der Temperatur des Dauerverformungsmittels die Körperwärme der zu behandelnden Person verwendet wird.

3. Verfahren nach Anspruch 1, dadurch gekennzeichnet, daß die zur Viskositätserniedriung des Dauerverformungsmittels erforderliche Temperaturerhöhung mittels Wärmestrahlung oder durch Aufblasen von erwärmter Luft bewirkt wird.

4. Verfahren nach einem der Ansprüche 1 bis 3, dadurch gekennzeichnet, daß ein Dauerverformungsmittel verwendet wird, bei dem der bzw. die konsistenzgebende(n) Zusatzstoff(e) in an sich bekannter Weise einen oder mehrere Paraffin(e), Petrolatum (Vaseline®), Ceresin(e), Fettalkohol(e), Fettsäure(n), Wachs(e), Polyglykol(e), Polyglykolester, Polyglykoläther, Aminoxid(e), Lanolinderivat(e) und/oder Ester und Äther ein- oder mehrwertiger organischer Säuren mit ein- oder mehrwertigen Alkoholen enthält bzw. enthalten.

**Claims**

1. Method for the permanent shaping of human hair, in which the hair rolled in portions onto curlers is treated with a hair keratin reducing permanent shaping agent containing a viscosity increasing additive or additives, which at room temperature (20°C) has a viscosity of at least 100 mPa/s, and which, after a sufficient time of action, is rinsed from the hair and the hair is then treated with a solution having an oxidizing action, characterized in that only one permanent shaping agent is used, which upon warming to a temperature within a temperature range between 25°C and 42°C undergoes a decided viscosity reduction of more than 60 %, with respect to the initial viscosity, and to a value under 50 mPa/s, that the permanent shaping agent is applied only to the portions of the hair wound without permanent shaping agent and lying outside in the curls, and that the viscosity of the permanent shaping agent is then reduced by elevating the temperature in a range between 25° and 42°C to such an extent that it becomes fluid and penetrates into the wound hair portions and wets them through.

2. Method in accordance with claim 1, characterized in that the body heat of the person to be treated is used for the elevation of the temperature of the permanent shaping agent.

3. Method in accordance with claim 1, characterized in that the temperature rise necessary for lowering the viscosity of the permanent shaping agent is produced by thermal radiation or by blowing heated air.

4. Method in accordance with any one of claims 1 to 3, characterized in that a permanent shaping agent is used in which the viscosity increasing additive or additives contain, in a known manner, one or more paraffins, petrolatum (Vaseline®), ceresins, fatty alcohols, fatty acids, waxes, polyglycols, polyglycol esters, polyglycol ethers, aminoxides, lanolin derivatives, and/or esters and ethers of monovalent or polyvalent organic acids with monovalent or polyvalent alcohols.

**Revendications**

1. Procédé pour le formage permanent de la chevelure dans lequel on traite les cheveux enroulés par parties sur des rouleaux par un produit de formage permanent réducteur de la kératine des cheveux et contenant des additifs conférant la consistance, ayant à température ambiante (20°C) une viscosité d'au moins 100 mPa.s et qui, après une durée d'action suffisante sur la chevelure, est rincé, après quoi la chevelure est traitée par une solution oxydante, caractérisé par le fait qu'on utilise un seul produit de formage permanent qui, chauffé à une température dans l'intervalle de 25 à 42°C, présente une diminution nette de viscosité, supérieure à 60 % par rapport à la viscosité initiale, laquelle tombe alors à une valeur inférieure à 50 mPa.s, que ce produit de formage permanent est appliqué uniquement sur les parties de la chevelure à l'extérieur des rouleaux, la chevelure ayant été mise sur rouleaux sans application de produit de formage permanent, que la viscosité du produit de formage permanent est ensuite abaissée par chauffage à une température dans l'intervalle de 25 à 42°C, au point qu'il devienne fluide et pénètre et mouille complètement les parties de la chevelure enroulées.

2. Procédé selon la revendication 1, caractérisé par le fait que le réchauffage du produit de formage permanent est provoqué par la chaleur corporelle de la personne dont on traite la chevelure.

3. Procédé selon la revendication 1, caractérisé par le fait que l'augmentation de température nécessaire pour abaisser la viscosité du produit de formage permanent est provoquée par des rayonnements calorifiques ou par soufflage d'air chaud.

4. Procédé selon l'une des revendications 1 à 3, caractérisé par le fait que l'on utilise un produit de formage permanent dans lequel le ou les additifs conférant la consistance contiennent, de manière connue en soi, une ou plusieurs paraffines, du pétrolatum (vaseline), de la cérésine, des alcools gras, des acides gras, des cires, des polyglycols, des esters de polyglycols, des éthers de polyglycols, des oxydes d'amines, des dérivés de la lanoline et/ou des esters et éthers d'acides organiques mono- ou poly-valents et d'alcools mono- ou poly-valents.